Europäisches Patentamt

European Patent Office

Office européen des brevets

(19)

(11) Veröffentlichungsnummer: **0 029 954**
**B1**

(12) # EUROPÄISCHE PATENTSCHRIFT

(45) Veröffentlichungstag der Patentschrift:
14.08.85

(21) Anmeldenummer: 80107159.8

(22) Anmeldetag: 18.11.80

(51) Int. Cl.⁴: **A 41 H 1/00**, G 01 B 11/24

(54) **Verfahren und Vorrichtung zur Ermittlung der menschlichen Körpermasse für Bekleidungszwecke auf photographischem Wege.**

(30) Priorität: 29.11.79 DE 2948010
02.06.80 DE 3020901

(43) Veröffentlichungstag der Anmeldung:
10.06.81 Patentblatt 81/23

(45) Bekanntmachung des Hinweises auf die Patenterteilung:
14.08.85 Patentblatt 85/33

(84) Benannte Vertragsstaaten:
AT BE CH FR GB IT LI NL SE

(56) Entgegenhaltungen:
DE - A - 1 034 375
GB - A - 855 101

THE BRITISH JOURNAL OF PHOTOGRAPHY, Band 12,
25. März 1977, London, GB, A.R. WILLIAMS:
"Techniques in applied photography; contour mapping
by light-sectioning", Seiten 265-266
ENGINEERING, 6. November 1970, London, GB, A.
SCOTT: "Photography can help", Seiten 494-495

(73) Patentinhaber: Landwehr, Ulrich M., Buersche Strasse 4,
D-4520 Melle 1 (DE)

(72) Erfinder: Landwehr, Ulrich M., Buersche Strasse 4,
D-4520 Melle 1 (DE)

ACTORUM AG

## Beschreibung

Die Erfindung betrifft ein Verfahren zur Ermittlung der menschlichen Körpermasse für Bekleidungszwecke auf photographischem Wege, bei dem eine Photographie der zu bekleidenden Person in verschiedenen Stellungen zusammen mit einem Massraster hergestellt wird, indem in einer ersten Aufnahme auf der einen Hälfte der Photographie die zu bekleidende Person in einer ersten Stellung und auf der anderen Hälfte das Massraster dargestellt werden, sodann das Massraster und die zu bekleidende Person in ihren Positionen vertauscht werden, wobei die zu bekleidende Person in einer zweiten Stellung angeordnet wird und in dieser Anordnung eine zweite Aufnahme der ersten Aufnahme überlagert wird.

Ein solches Verfahren ist aus der GB-A-855101 bekannt.

Bei einem weiter bekannten Verfahren (DE-PS 1 034 375) wird eine Photographie der zu bekleidenden Person vor einer mit einer Markierungslinie versehenen Platte hergestellt, wobei an die Person Massbänder angelegt sind. Nach erfolgter Aufnahme wird die Platte gewendet, die nunmehr ein Massraster zeigt und so weit verschoben, bis ihre Fläche genau mit der auf dem Boden markierten Linie zusammenfällt, die die mittlere Schnittebene der Person zeigt, die vorher dort gestanden hat. Sodann wird bei unveränderter Kamerastellung durch nochmalige Belichtung das Massraster auf dieselbe Platte oder denselben Film über das Bild der vorher aufgenommenen Person photographiert. Man erhält somit ein Photo der Person mit einem überlagerten Massraster. In derselben Weise wird die Person in drei verschiedenen Richtungen aufgenommen.

Diese Vorgehensweise ist zeitlich recht aufwendig, da für jede Ansicht mindestens zwei Aufnahmen gemacht werden müssen, d.h. für die Rückenpartie und eine Seitenansicht vier Aufnahmen. Darüber hinaus stellt das Verschieben der Platte hohe Anforderungen an die Sorgfalt des Bedienungspersonals und ist recht zeitaufwendig. Insbesondere aber ist dieses Verfahren nicht geeignet, körperliche Mängel, wie Wirbelsäulenverkrümmung, hervorstehendes Schulterblatt u. ä. zu erfassen.

Zur Vermessung einer dreidimensionalen Oberfläche ist auch das Lichtschnittverfahren bekannt, wobei ein Muster von gleichbeabstandeten parallel zueinander verlaufenden Linien auf die Oberfläche projiziert wird (vgl. die Zeitschriften «The British Journal of Photography» Band 12, 25. März 1977, Seiten 265 und 266, sowie «Engineering» 6. November 1970, Seiten 494 und 495).

Der vorliegenden Erfindung liegt von daher die Aufgabe zugrunde, ein Verfahren anzugeben, mit dem die Ermittlung der menschlichen Körpermasse wesentlich einfacher und schneller durchgeführt werden kann. Insbesondere sollen auch Wölbungen der Oberfläche des Körpers genau messbar werden.

Diese Aufgabe wird bei einem Verfahren der eingangs erwähnten Art dadurch gelöst, dass erfindungsgemäss während der Aufnahmen ein unter einem Winkel von 30–60° zur Vertikalen von oben einfallendes Muster aus horizontal verlaufenden, in der Massrasterebene äquidistanz zueinander angeordneten Linien auf die Person projiziert wird.

Das Verfahren gemäss der Lehre der Erfindung benötigt für zwei Ansichten lediglich eine aus zwei Belichtungen hergestellte Photographie und ist somit wesentlich wirtschaftlicher als das bekannte Verfahren. Der wesentliche Vorteil ist jedoch darin zu sehen, dass durch das auf die Person projizierte Linienmuster erhabene und einfallende Körperpartien sichtbar und messbar gemacht werden können, d.h. die dritte Dimension kenntlich gemacht werden kann. Massfaktoren sind Abstand der Linien sowie Krümmungsrichtung der Linien.

Insbesondere, wenn das Linienmuster jeweils mittig auf den Gegenstand bzw. auf die zu bekleidende Person projiziert wird, lässt sich aus dem Linienverlauf und dem Linienabstand ein genauer Verlauf der Oberfläche des Körpers bzw. der Person ermitteln. Mit besonderem Vorteil wird das Linienmuster mittels eines mit dem Blitzlicht der Kamera photoelektrisch gekoppelten Blitzlichtprojektor projiziert. Obwohl nahezu jeder Winkel zwischen 0 und 90° zur Vertikalen möglich ist, hat es sich als zweckmässig erwiesen, das Linienmuster unter einem Winkel von 30 bis 60° zur Vertikalen auf den Körper oder die Person zu projizieren. In diesem Bereich lassen sich Unebenheiten besonders deutlich darstellen. Das Linienmuster wird so auf den Körper bzw. die Person projiziert, dass die Linien auf eine vertikale Wand projiziert den gleichen Abstand zueinander aufweisen. Verzerrungen sind dadurch ausgeschaltet. Bei Personen hat es sich als vorteilhaft erwiesen, für Bekleidungszwecke lediglich die Rückenpartie und eine Seitenpartie zu photographieren.

Das erfindungsgemässe Verfahren ermittelt die Abmessungen so genau, dass auf eine Abbildung der Frontpartie im Normalfall verzichtet werden kann.

Die Erfindung betrifft weiterhin eine Vorrichtung zur Durchführung des Verfahrens, die sich von der bekannten Vorrichtung dadurch unterscheidet, dass zwischen Kamera und Platte schräg oberhalb der zu photographierenden Person ein das Linienmuster projizierender Blitzlichtprojektor angeordnet ist. Zweckmässigerweise ist die Platte um eine vertikale Achse um mindestens 180° schwenkbar, ist die Kamera auf die Schwenkachse ausgerichtet und die Platte in beiden Stellungen auf der Photographie darstellbar. Für diese Vorrichtung gelten neben den oben bereits genannten Vorteilen, dass sie einfach im Aufbau ist und auch von nicht geschultem Personal bedient werden kann.

Zweckmässigerweise ist mit Blitzlichtprojektor ein das Linienmuster tragendes Diapositiv angeordnet. Das Diapositiv kann ggf. schnell gegen ein anders geartetes Linienmuster ausgetauscht werden.

Mit besonderem Vorteil ist der Blitzlichtprojektor parallel zur Kamera ausgerichtet, und zwar insbesondere in Photographierstellung jeweils mittig auf die Platte. Dadurch ergeben sich keinerlei optische Verzerrungen und die Oberfläche des Körpers bzw. der Person wird optimal dargestellt.

Zwecks leichteren Transports des Blitzlichtprojektors in seine beiden Photographierstellungen ist dieser parallel zur Platte verfahrbar. Hierzu dient eine Schiene, an welcher der Blitzlichtprojektor über Rollen angehängt ist.

Der Blitzlichtprojektor ist nach einer besonders günstigen Ausgestaltung der Vorrichtung mit der Platte gekoppelt und beim Schwenken der Platte und das Verfahren des Blitzlichtprojektors ist somit lediglich ein Handgriff erforderlich. Darüber hinaus wird durch diese Ausführungsform gewährleistet, dass der Blitzlichtprojektor nach dem Schwenken der Platte exakt in Photographierstellung befindlich ist.

Für einen geräuscharmen und ruckfreien Transport des Blitzlichtprojektors sorgt ein über Rollen geführtes endloses Band, an welches der Blitzlichtprojektor angekoppelt ist und ein am vom Blitzlichtprojektor entferntesten Ende des Bandes angelenktes Teleskoprohr, welches ebenfalls gelenkig mit der Platte verbunden ist.

Diese Konstruktion ist für manche Anwendungsfälle störanfällig und kann nicht gewährleisten, dass das Liniemuster immer von der gleichen Stellung aus projiziert wird. Bei vergleichenden Messungen, zum Beispiel für die Messung der Veränderung von Körpermassen, ist diese Vorrichtung noch nicht zufriedenstellend.

Nach einem weiteren Gedanken der Erfindung ist deshalb vorgesehen, dass für jede Aufnahme der beiden Stellungen der Person ein gesonderter, fest installierter, auf einen bestimmten Punkt ausgerichteter Blitzlichtprojektor, der das Linienmuster während der Aufnahme projiziert, vorgesehen ist. Dadurch kann der Einfallwinkel des Linienmusters konstant gehalten werden, und somit ein genaues Messergebnis ermöglicht werden.

Damit eine optische Verzerrung des Linienrasters vermieden ist und auf einer vertikalen Fläche der Abstand der projizierten Linien gleich ist, besitzt der Blitzlichtprojektor ein korrigiertes optisches Linsensystem, in welchem die der Blitzlichtquelle zugekehrte Linse des Systems zur Achse des Systems geneigt ist.

Nach einer weiteren besonders vorteilhaften Ausgestaltung der Vorrichtung gemäss der Lehre der Erfindung zeigt das mit dem Linienmuster versehene Diapositiv mehrere vertikal verlaufende Punktreihen mit mindestens einem zwischen jeweils zwei Linien angeordneten Punkt, wobei der Abstand der Punktreihen zueinander mindestens dem doppelten höchstens aber dem 5-fachen Linienabstand entspricht. Beim Auftreffen auf eine schräge Fläche der Person wird nicht mehr ein Punkt, sondern eine vertikal verlaufende Linie erzeugt, welche die Darstellung der Oberfläche des Körpers bzw. der Person noch genauer macht. Der beanspruchte Abstand der Punktreihen ist völlig ausreichend, ohne das Bild zu verwirren.

Die Erfindung ist anhand der in den Figuren 1 bis 4 schematisch dargestellten Ausführungsbeispiele näher erläutert. Es zeigen

Figur 1 eine nach dem erfindungsgemässen Verfahren hergestellte Photographie;

Figur 2 eine Draufsicht auf die Vorrichtung zur Herstellung der Photographie;

Figur 3 eine Seitenansicht der Vorrichtung und

Fig. 4 eine Draufsicht auf eine abgewandelte Vorrichtung.

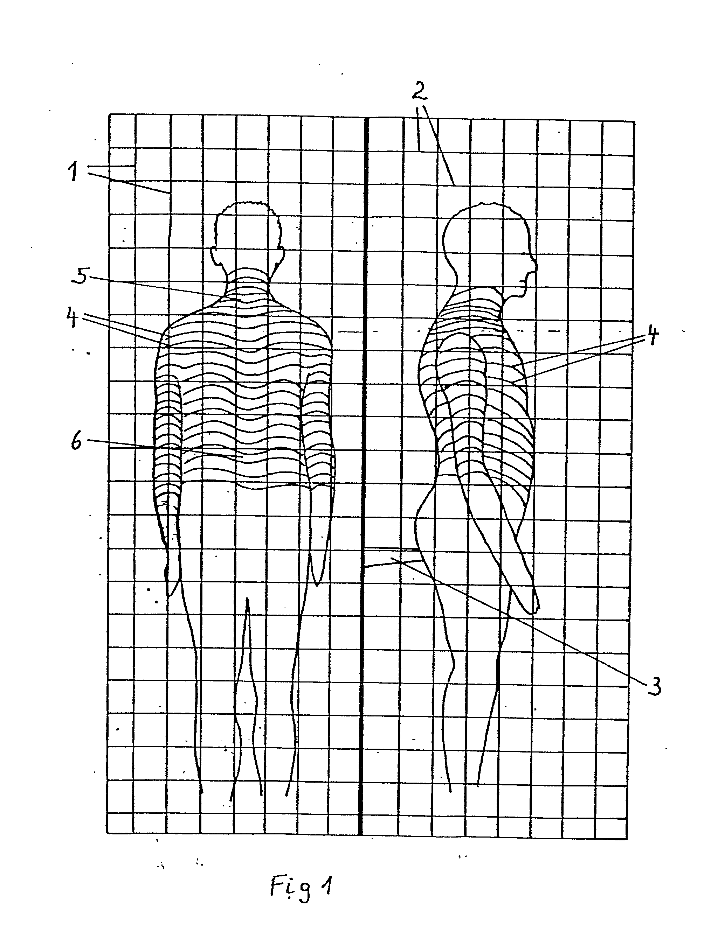

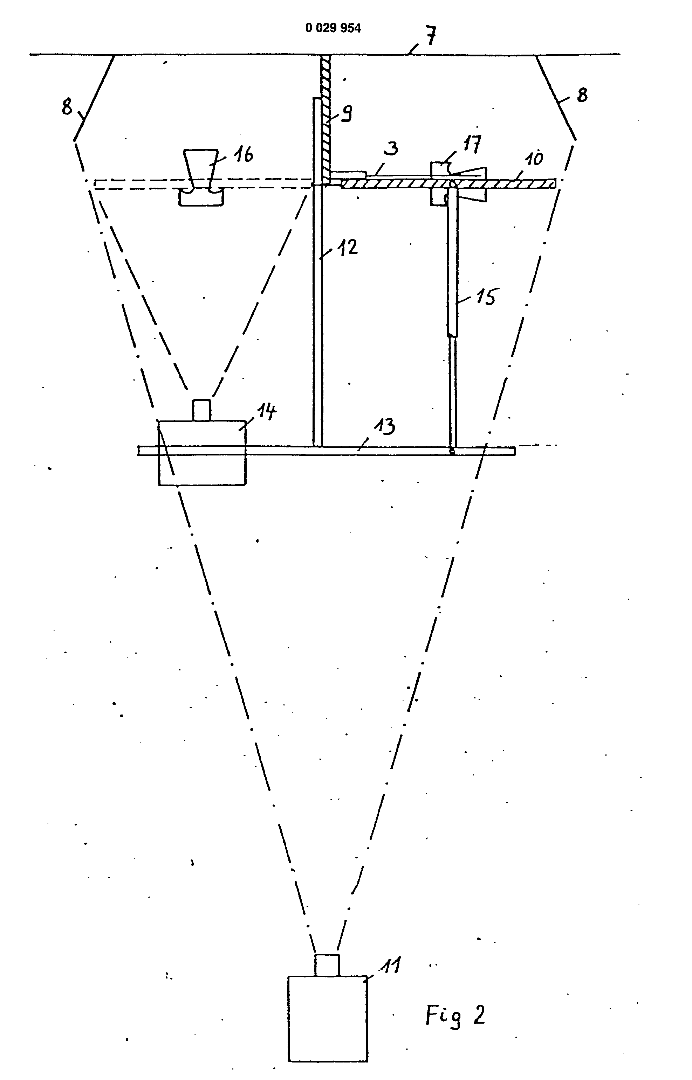

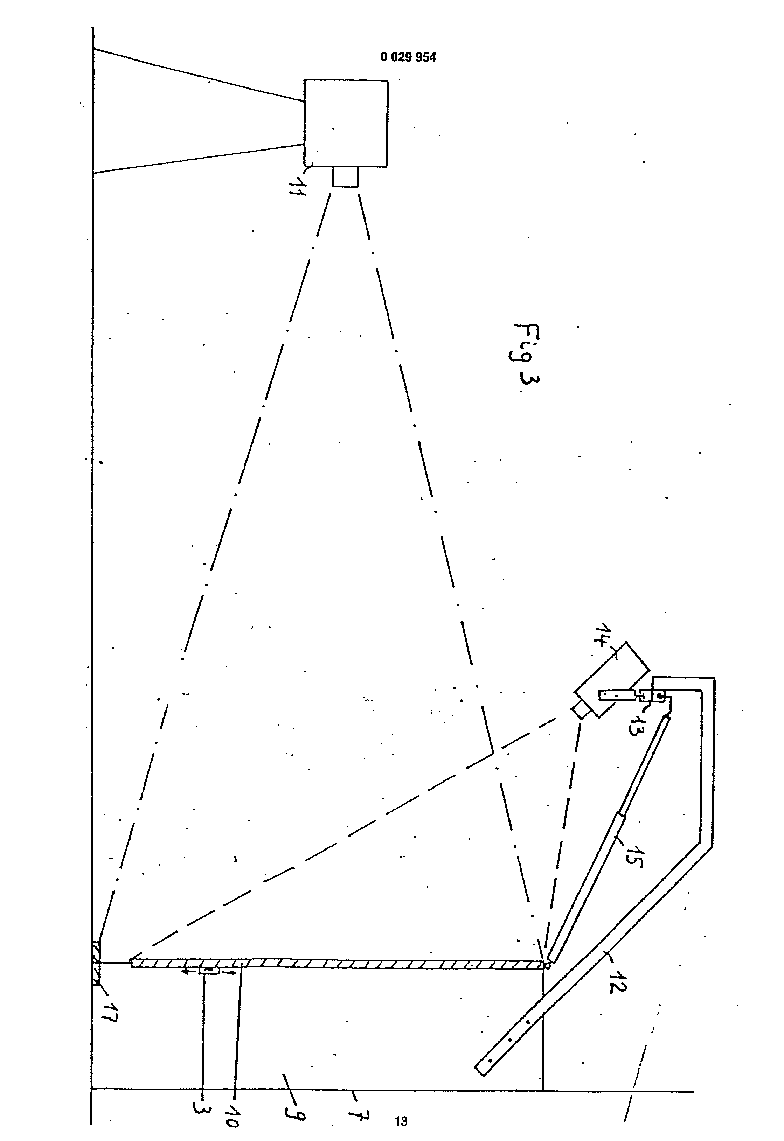

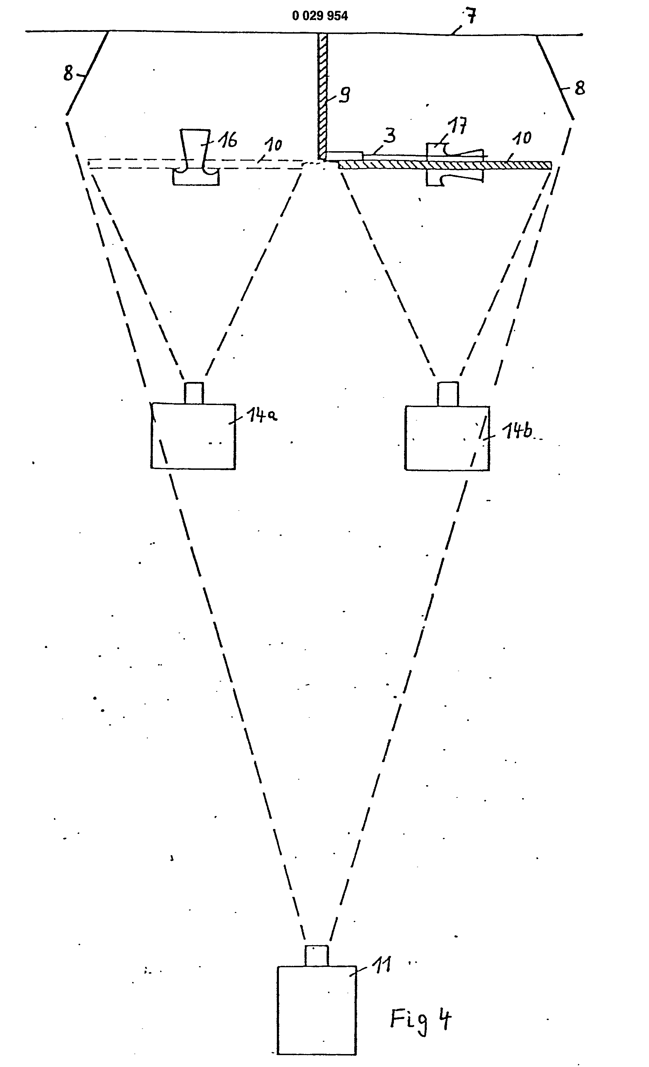

Auf der Photographie (s. Figur 1) ist in der linken Hälfte eine Person von hinten und in der rechten Hälfte eine Person in Seitenansicht dargestellt. Beide Hälften der Photographie zeigen ein Massraster aus vertikalen Linien 1 und horizontalen Linien 2, deren Abstand zueinander mit 1 cm festgelegt ist. Zur einfacheren Erfassung der Abmasse sind alle 10 cm die Masse seitlich aufgeführt. Ein höhenverstellbares schwenkbares Lineal 3 ermöglicht das schnelle Ablesen der Schrittlänge.

Ein Linienmuster aus im wesentlichen horizontal verlaufenden Linien 4 verdeutlicht durch unterschiedliche Krümmungen und Linienabstände die Kontur der Oberfläche. So zeigt beispielsweise der kürzere Abstand an der mit 5 bezeichneten Stelle den geneigten Verlauf der Nackenpartie, während der Verlauf der Senken der Linien bei 6 auf eine leichte Verkrümmung der Wirbelsäule schliessen lässt. Die Auswertung der Photographie ermöglicht somit die Herstellung von körpergerechten Schnittmustern bzw. ermöglicht die Photographie die passgenaue Herstellung von Zuschnitten für die herzustellende Bekleidung. Die Photographie zeigt die genaue Rückenform, wie sie bislang nicht messbar war und macht die dritte Dimension deutlich.

In der Figur 2 ist mit 7 eine Wand bezeichnet, an welcher lichtreflektierende Spiegel 8 angebracht sind. Nahezu in der Mitte zwischen den Spiegeln 8 ist eine lotrechte Standplatte 9 mechanisch fest an der Wand 7 angebracht. Das Lineal 3 ist in Richtung der Horizontalen schwenkbar sowie höhenverstellbar. Am freien Ende der Standplatte 9 ist zum Beispiel über ein nicht näher bezeichnetes Scharnierband eine Platte 10 befestigt, welche um 180° schwenkbar ist. Die Platte 10 trägt auf beiden Seiten das aus den Linien 1 und 2 gebildete Massraster. Eine Kamera 11, welche mit einem Blitzlicht ausgestattet ist und vorzugsweise eine Sofortbildkamera ist, befindet sich im Abstand von ca. 3 m von der Platte 10 und ist auf die Schwenkachse der Platte 10 ausgerichtet. An der Standplatte 9 ist eine Halterung 12 befestigt, die an ihrem freien Ende eine Laufschiene 13 trägt, die exakt horizontal ausgerichtet ist. An dieser Laufschiene 13 hängt über nicht dargestellte Rollen ein Blitzlichtprojektor 14, dessen Blitzlicht mit dem Blitzlicht der Kamera 11 gleichgeschaltet ist. Der Blitzlichtprojektor 14 ist in nicht dargestellter Weise an einem über Rollen geführten Band befestigt, das bei Schwenkung der Platte 10 bewegt wird. Hierzu ist die Platte 10 an ein Teleskopgestänge 15 drehbar angelenkt, dessen anderes Ende ebenfalls drehbar an dem Band befestigt wird. Wird nun die Platte 10 geschwenkt, schiebt sich zunächst das Teleskopgestänge 15 etwas zusammen. Durch die Kraftkomponente in Richtung senkrecht zur Halterung 12 wird das Band in Bewegung versetzt, wodurch der Blitzlichtprojektor in der Figur von links nach rechts wandert. Die Platte 10 nimmt am Ende des Schwenkvorgangs die gestrichelt dargestellte Position ein. Der Blitzlichtprojektor 14 ist in seinen beiden Endstellungen jeweils auf die Mitte der Platte 10 ausgerichtet, wenn diese um 180° geschwenkt wäre. Der Blitzlichtprojektor 14 projiziert mittels eines nicht näher bezeichneten Diapositivs ein Linienmuster auf die Person. Um eine Verzerrung des Linienmusters zu vermeiden, ist ein spezielles Linsensystem im Blitzlichtprojektor 14 vorgesehen, dessen der Wand 7 abgekehrte Linse zur Achse des optischen Systems geneigt ist. Der Blitzlichtprojektor 14 ist so eingestellt, dass sein Lichtkegel im Bereich der Platte 10 der Breite der Platte 10 entspricht. Der Aufnahmebereich der Kamera 11 erstreckt sich im wesentlichen über die doppelte Breite der Platte 10. Mit 16 und 17 sind Fussrasten bezeichnet, in welche eine zu photographierende Person vor der Aufnahme eintritt.

Die Figur 3 verdeutlicht die Anordnung in der Seitenansicht. Hieraus ist ersichtlich, dass der Blitzlichtprojektor 14 zwischen der Platte 10 und der Kamera 11 unter einem Winkel von ca. 45° auf die Ebene der Platte 10 ausgerichtet ist, jedoch so, dass das von ihm projizierte Linienraster im Bereich der Ebene der Platte 10 zumindest die gesamte Höhe der Platte 10 abdeckt (s. gestrichelte Linien). Das Gleiche gilt für den Aufnahmebereich der Kamera 11 (s. strichpunktierte Linien).

Das erfindungsgemässe Verfahren arbeitet wie folgt: Zunächst wird – wie in der Figur 2 – eine Photographie gemacht, wobei die Person in den Fussrasten 16 steht. Gleichzeitig mit dem Blitzlicht der Kamera 11 wird das Blitzlicht des Blitzlichtprojektors 14 photoelektrisch ausgelöst. Auf der Photographie ist nun in der linken Hälfte die Person mit dem auf ihr projizierten Linienmuster und in der rechten Hälfte das Massraster der Platte 10 dargestellt. Nun wird die Platte 10 bis in die gestrichelt dargestellte Position geschwenkt. Dabei wandert, wie oben beschrieben, der Blitzlichtprojektor 14 entlang der Schiene 13 von links nach rechts, und das Teleskopgestänge 15 von rechts nach links. Die Person begibt sich in die Fussrasten 17, und durch eine weitere Belichtung der in der Kamera 11 befindlichen Platte bzw. des Films wird auf die bei der ersten Belichtung abgelichteten Massraster die Person in Seitenansicht überlagert, wobei auch auf die Seitenansicht das Linienmuster während der Aufnahme projiziert wird.

Mit Hilfe des erfindungsgemässen Verfahrens ist es möglich geworden, in kürzester Zeit eine weitestgehend massgenaue Aufnahme von zum Beispiel der Rückenpartie und einer Seitenansicht herzustellen, die die Oberfläche der Person einwandfrei darstellt. Mit besonderem Vorteil lassen sich die nach dem Verfahren gemäss der Lehre der Erfindung erstellten Abbildungen zur Herstellung von Oberbekleidung wie Sakkos, Hosen, Mäntel etc. verwenden.

Das Verfahren ist nicht auf den anhand der Figurenbeschreibung hervorgehobenen Anwendungsfall der Herstellung von Abbildungen zum Zwecke der Bekleidungsherstellung beschränkt, sondern ist auch geeignet zur Ermittlung der Abmessungen eines Gegenstandes.

Eine besonders günstige Vorrichtung ist in der Figur 4 dargestellt. Im Gegensatz zu dem verfahrbaren Blitzprojektor 14 sind zwei Blitzprojektoren fest installiert. Das Blitzlicht der Kamera 11 ist photoelektrisch gekoppelt mit je einem der Blitzprojektoren 14a oder 14b. Die Blitzprojektoren 14a und 14b sind unter einem bestimmten Winkel von oben auf die Ebene der Platte 10 ausgerichtet. Die Blitzprojektoren projizieren auf die in den Fussrasten 16 bzw. 17 stehende Person das Linienmuster, welches durch ein Diapositiv erzeugt wird. Die Linienabstände auf dem Diapositiv sind entsprechend dem Blitzprojektor-Neigungswinkel so errechnet, dass die auf die schräge Ebene projiziert auffallenden Linien äquadistant sind. Die Blitzprojektoren 14a und 14b werden wechselseitig über einen nicht dargestellten, an der Platte 10 angebrachten Kontakt geschaltet. Steht die Person an der Fussraste 17, wird der Blitzlichtprojektor 14b, steht die Person in der Fussraste 16, wird der Blitzlichtprojektor 14a betätigt.

Der wesentliche Vorteil dieser Vorrichtung besteht darin, dass mit einfachen Mitteln sichergestellt ist, den Einfallwinkel des Linienmusters konstant zu halten und somit die dritte Dimension der Person, d.h. Wölbungen etc. sichtbar und exakt messbar zu machen.

**Patentansprüche**

1. Verfahren zur Ermittlung der menschlichen Körpermasse für Bekleidungszwecke auf photographischem Wege, bei dem eine Photographie der zu bekleidenden Person in verschiedenen Stellungen zusammen mit einem Massraster hergestellt wird, indem in einer ersten Aufnahme auf der einen Hälfte der Photographie die zu bekleidende Person in einer ersten Stellung und auf der anderen Hälfte das Massraster dargestellt werden, sodann das Massraster und die zu bekleidende Person in ihren Positionen vertauscht werden, wobei die zu bekleidende Person in einer zweiten Stellung angeordnet wird und in dieser Anordnung eine zweite Aufnahme der ersten Aufnahme überlagert wird, dadurch gekennzeichnet, dass während der beiden Aufnahmen ein unter einem Winkel von 30–60° zur Vertikalen von oben einfallendes Muster aus horizontal verlaufenden, in der Massrasterebene äquidistant zueinander angeordneten Linien auf die Person projiziert wird.

2. Verfahren nach Anspruch 1, dadurch gekennzeichnet, dass das Linienmuster jeweils mittig auf die Person projiziert wird.

3. Verfahren nach Anspruch 1 oder 2, dadurch gekennzeichnet, dass das Linienmuster mittels eines mit dem Blitzlicht der Kamera photoelektrisch gekoppelten Blitzlichprojektor projiziert wird.

4. Verfahren nach Anspruch 1 oder einem der folgenden, dadurch gekennzeichnet, dass die zu bekleidende Person von hinten und von der Seite abgelichtet wird.

5. Vorrichtung zur Durchführung des Verfahrens nach Anspruch 1 oder einem der folgenden, bestehend aus einer Platte, welche mit einem Massraster versehen ist, wowie einer in bestimmtem Abstand zur Platte installierten Kamera, dadurch gekennzeichnet, dass zwischen Kamera (11) und Platte (10) schräg oberhalb der zu photographierenden Person mindestens ein das Linienmuster projizierender Blitzlichprojektor (14) angeordnet ist, wobei Kamera (11) und Blitzlichprojektor (14) frontal zur Plattenebene angeordnet sind.

6. Vorrichtung nach Anspruch 5, dadurch gekennzeichnet, dass die Platte (10) um eine vertikale Achse um mindestens 180° schwenkbar ist, dass die Kamera (11) auf die Schwenkachse ausgerichtet ist und die Platte (10) in beiden Schwenkstellungen auf der Photographie darstellbar ist.

7. Vorrichtung nach Anspruch 5 oder 6, dadurch gekennzeichnet, dass im Blitzlichprojektor (14) ein das Linienmuster tragendes Diapositiv angeordnet ist.

8. Vorrichtung nach Anspruch 5 oder einem der folgenden, dadurch gekennzeichnet, dass der Blitzlichprojektor (14) parallel zur Kamera (11) ausgerichtet ist, wobei der Blitzlichprojektor unter einem Winkel von 30–60° zur Vertikalen angeordnet ist.

9. Vorrichtung nach Anspruch 5 oder einem der folgenden, dadurch gekennzeichnet, dass der Blitzlichprojektor (14) in Photographierstellung jeweils mittig auf die Platte (10) ausgerichtet ist.

10. Vorrichtung nach Anspruch 5 oder einem der folgenden, dadurch gekennzeichnet, dass der Blitzlichprojektor (14) parallel zur Platte (10) verfahrbar ist.

11. Vorrichtung nach Anspruch 5 oder einem der folgenden, dadurch gekennzeichnet, dass der Blitzlichprojektor (14) an einer Schiene (13) über Rollen verfahrbar ist.

12. Vorrichtung nach Anspruch 5 oder einem der folgenden, dadurch gekennzeichnet, dass der Blitzlichprojektor (14) mit der Platte (10) gekoppelt ist und beim Schwenken der Platte (10) entlang der Schiene (13) verfahrbar ist.

13. Vorrichtung nach Anspruch 5 oder einem der folgenden, dadurch gekennzeichnet, dass entlang der Schiene (13) ein über Rollen geführtes endloses Band angeordnet ist, an welches der Blitzlichprojektor (14) angekoppelt und dass am vom Blitzlichprojektor (14) entferntesten Ende des Bandes ein gelenkig mit der Platte (10) verbundenes Teleskoprohr (15) angelenkt ist.

14. Vorrichtung nach Anspruch 5 oder einem der folgenden, dadurch gekennzeichnet, dass für jede Aufnahme der beiden Stellungen des Gegenstandes oder der Person ein gesonderter, fest installierter, auf einen bestimmten Punkt ausgerichteter Blitzlichprojektor (14a, 14b), der das Linienmuster während der Aufnahme projiziert, vorgesehen ist.

15. Vorrichtung nach Anspruch 5 oder einem der folgenden, dadurch gekennzeichnet, dass der Blitzlichprojektor (14) ein korrigiertes optisches Linsensystem aufweist, in dem die der Blitzlichtquelle zugekehrte Linse des Systems zur Achse des Systems geneigt ist.

16. Vorrichtung nach Anspruch 5 oder einem der folgenden, dadurch gekennzeichnet, dass das mit dem Linienmuster versehene Diapositiv vertikal verlaufende Punktreihen mit mindestens einem zwischen jeweils zwei Linien angeordneten Punkt zeigt, deren Abstand zueinander mindestens dem doppelten Linienabstand höchstens aber dem 5-fachen Linienabstand entspricht.

## Claims

1. Process for determining human body measurements photographically for clothing purposes, in which a photograph of the person to be clothed is taken in different positions, together with a measurement grid, wherein, in a first shot, the person to be clothed is represented in a first position on one half of the photograph and the measurement grid is represented on the other half, the positions of the measuring grid and of the person to be clothed are then interchanged, the person to be clothed is arranged in a second position, and in this arrangement a second shot is superimposed on the first shot, characterised in that during the two shots a pattern coming from above at an angle of 30 to 60° relative to the vertical and consisting of horizontal lines equidistant from one another in the plane of the measurement grid is projected onto the person.

2. Process according to the Claim 1, characterised in that each time the line pattern is projected centrally onto the person.

3. Process according to Claim 1 or 2, characterised in that line pattern is projected by means of a flash light projector coupled photoelectrically to the flash light of the camera.

4. Process according to Claim 1 or one of the following Claims, characterised in that the person to be clothed is photographed from the rear and from the side.

5. Apparatus for carrying out the process according to Claim 1 or one of the following Claims, consisting of a plate provided with a measurement grid and of a camera installed at a specific distance from the plate, characterised in that at least one flash light projector (14) projecting the line pattern is arranged obliquely above the person to be photographed, between the camera (11) and the plate (10), the camera (11) and flash light projector (14) being arranged in front of the plate plane.

6. Apparatus according to Claim 5, characterised in that the plate (10) is pivotable at least 180° about a vertical axis, in that the camera (11) is aligned with the pivot axis and the plate (10) can be represented on the photograph in the two pivoting positions.

7. Apparatus according to Claim 5 or 6, characterised in that a transparency carrying the line pattern is arranged in the flash light projector (14).

8. Apparatus according to Claim 5 or one of the following Claims, characterised in that the flash light projector (14) is aligned parallel to the camera (11), the flash light projector being arranged at an angle of 30 to 60° to the vertical.

9. Apparatus according to Claim 5 or one of the following Claims, characterised in that, in each of the photographing positions, the flash light projector (14) is aligned centrally relative to the plate (10).

10. Apparatus according to Claim 5 or one of the following Claims, characterised in that the flash light projector (14) is movable parallel to the plate (10).

11. Apparatus according to Claim 5 or one of the following Claims, characterised in that the flash light projector (14) is movable on a rail (13) by means of rollers.

12. Apparatus according to Claim 5 or one of the following Claims, characterised in that the flash light projector (14) is coupled to the plate (10) and, when the plate (10) is pivoted, is movable along the rail (13).

13. Apparatus according to Claim 5 or one of the following Claims, characterised in that arranged along the rail (13) is an endless belt which is guided by means of rollers, to which the flash light projector (14) is coupled, and in that a telescopic tube (15) connected pivotally to the plate (10) is articulated on the end of the belt located furthest away from the flash light projector (14).

14. Apparatus according to Claim 5 or one of the following Claims, characterised in that for each shot of the two positions of the article or the person there is a separate, fixedly installed flash light projector (14a, 14b) which is aligned with a specific point and projects the line pattern during the shot.

15. Apparatus according to Claim 5 or one of the following Claims, characterised in that the flash light projector (14) has a corrected optical lens system, in which the system lens facing the flash light source is inclined relative to the axis of the system.

16. Apparatus according to Claim 5 or one of the following Claims, characterised in that the transparency provided with the line pattern has vertically extending rows of points, with at least one point located between any two lines, the distance between them corresponding to at least double the distance between the lines, but at most to five times the distance between the lines.

**Revendications**

1. Procédé pour déterminer par des moyens photographiques les dimensions du corps humain en vue de la confection de vêtements, procédé dans lequel on réalise une photographie, comportant en même temps une grille de mesures prise dans différentes positions, de la personne à habiller, procédé selon lequel, dans une première prise de vue, la personne à habiller est représentée dans une première position sur une des moitiés de la photographie, tandis que la grille de mesures est représentée sur l'autre moitié, puis les emplacements de la grille de mesures et de la personne à habiller sont permutés, la personne à habiller étant placée dans une seconde position, et dans cette situation, une seconde prise de vue est superposée à la première prise de vue, procédé caractérisé en ce que pendant les deux prises de vues, une trame constituée de lignes s'étendant horizontalement et équidistantes les unes des autres dans le plan de la grille de mesures, est projetée sur la personne à partir du haut sous un angle de 30 à 60° par rapport à la verticale.

2. Procédé selon la revendication 1, caractérisé en ce que la trame est chaque fois projetée en étant centrée sur la personne.

3. Procédé selon revendication 1 ou 2, caractérisé en ce que la trame est projetée au moyen d'un projecteur à éclair de lumière couplé photoélectriquement avec l'éclair de lumière de l'appareil photographique.

4. Procédé selon revendication 1 ou l'une des suivantes, caractérisé en ce que la personne à habiller est photographiée de l'arrière et de côté.

5. Dispositif pour la mise en œuvre du procédé selon la revendication 1 ou une des suivantes, dispositif constitué d'une plaque munie d'une grille de mesures, ainsi que d'un appareil photographique installé à une distance déterminée de cette plaque, dispositif caractérisé en ce que, entre l'appareil de mesure (11) et la plaque (10) au moins un projecteur à éclair de lumière (14), projetant la trame, est disposé obliquement au-dessus de la personne à photographier, l'appareil photographique (11) et la projecteur à éclair de lumière (14) étant disposés frontalement par rapport au plan de la plaque.

6. Dispositif selon la revendication 5, caractérisé en ce que la plaque (10) est susceptible de pivoter d'au moins 180° autour d'un axe vertical, l'appareil photographique (11) étant orienté vers cet axe de pivotement, et la plaque (10) étant susceptible d'être représentée sur la photographie dans ses deux positions de pivotement.

7. Dispositif selon revendication 5 ou 6, caractérisé en ce que, une diapositive portant la trame est disposée dans le projecteur à éclair de lumière (14).

8. Dispositif selon la revendication 5 ou une des suivantes, caractérisé en ce que le projecteur à éclair de lumière (14) est orienté parallèlement à l'appareil photographique (11), ce projecteur à éclair de lumière étant disposé selon un angle de 30 à 60° par rapport à la verticale.

9. Dispositif selon la revendication 5 ou une des suivantes, caractérisé en ce que les projecteur à éclair de lumière (14) dans la position de photographie est orienté chaque fois sur le milieu de la plaque (10).

10. Dispositif selon la revendication 5 ou une des suivantes, caractérisé en ce que le projecteur à éclair de lumière (14) est susceptible d'être déplacé parallèlement à la plaque (10).

11. Dispositif selon la revendication 5 ou une des suivantes, caractérisé en ce que le projecteur à éclair de lumière (14) est susceptible d'être déplacé sur un rail (13) par l'intermédiaire de galets.

12. Dispositif selon la revendication 5 ou une des suivantes, caractérisé en ce que le projecteur à éclair de lumière (14) est couplé avec la plaque (10), et qu'il est susceptible de se déplacer le long du rail (13) lorsque la plaque (10) pivote.

13. Dispositif selon la revendication 5 ou une des suivantes, caractérisé en ce qu'une bande sans fin guidée par l'intermédiaire de galets, est disposée le long du rail (13), le projecteur à éclair de lumière (14) étant accouplé à cette bande, tandis qu'à l'extrémité de la bande la plus éloignée du projecteur à éclair de lumière (14) est articulé un tube télescopique (15) relié par articulation à la plaque (10).

14. Dispositif selon la revendication 5 ou une des suivantes, caractérisé en ce que, pour chaque prise de vue, de l'une à l'autre position de l'objet ou de la personne, il est prévu un projecteur à éclair de lumière (14a, 14b), distinct, installé à poste fixe, dirigé sur un point déterminé, et qui projette la trame pendant la prise de vue.

15. Dispositif selon la revendication 5 ou une des suivantes caractérisé en ce que le projecteur à éclair de lumière (14) comporte un système optique de lentille corrigé, dans lequel la lentille du système tournée vers la source de l'éclair de lumière est inclinée par rapport à l'axe du système.

16. Dispositif selon la revendication 5 ou une des suivantes caractérisé en ce que la diapositive munie de la trame présente des rangées de points s'étendant verticalement avec au moins un point disposé entre respectivement deux lignes, la distance d'un point à l'autre correspondant au moins au double de la distance entre les lignes et au plus à cinq fois cette distance.

Fig 1

0 029 954

Fig 2

Fig 3

Fig 4